# EUROPEAN PATENT APPLICATION

(11) **EP 3 730 092 A1**
(43) Date of publication of application: **28.10.2020**
(21) Application number: 20171035.7
(22) Date of filing: 23.04.2020
(51) Int. Cl.: A61D 19/02, A61D 19/04, A61B 17/43, A61B 17/435

(54) **APPARATUS FOR ARTIFICIAL INSEMINATION OF MAMMALS AND METHOD FOR MAKING THE SAME**

(30) Priority: 23.04.2019 US 201916392374
(71) Applicant: Beachy, Bryan J., Jefferson, OR 97352 (US)
(72) Inventor: Beachy, Bryan J., Jefferson, OR 97352 (US)
(74) Representative: Laine IP Oy

(57) **Abstract**

In accordance with one aspect of the invention, an apparatus for depositing semen into a uterus of a mammal includes a nonlinear probe portion that is curved to approximate the angle between the vagina and cervix of a mammal. The probe has a caudal end that is flared to accept a stopping device, the stopping device preventing airflow that would cause the lens of a camera to become contaminated. The probe has a plunger that passes through the length of the probe that, when activated, deposits the semen into the uterus of the mammal through an opening in the distal end of the probe. The distal end of the probe is equal to, or less than, the penis of a male of the same species that is being inseminated.

## Description

### BACKGROUND OF THE INVENTION

### 1. Field of the Invention

The present invention relates to an apparatus and a method useful for non-surgical embryo transfer or artificial insemination of mammals. An apparatus for depositing media into the uterus of a mammal includes an artificial insemination transfer device for depositing media into a uterus, and a camera for guiding the device into a uterus. The invention also involves a method employing the devices describe.

### 2. Discussion of the Related Art

In recent years, effective application of artificial impregnation including artificial insemination and non-surgical embryo transfer has established a proven method for improving the production of domestic livestock. Generally, such techniques enhanced the ability to selectively breed a single genetically superior male for production traits with many females.

Selective breeding of course allows for livestock with improved genetic traits for production. Artificial insemination techniques also decrease the chance of diseases and physical injury that can be associated with the natural breeding process. As a result of these and other advantages, the use of artificial insemination and non-surgical embryo transfer have become a widespread technique in the management of many species of domestic livestock.

One of the non-surgical embryo transfer systems described in the prior art, involves inserting a tubular instrument into the cervix of a recipient female, and then depositing 10-12 milliliters of liquid medium containing embryos into and through the instrument, the objective being to deposit the embryos in the uterus. However, other procedures have several drawbacks. First, there is no way to determine whether the instrument has been inserted far enough into the cervix so that its forward end is adjacent to the body of the uterus. As a result, instances where the forward end of the instrument remains lodged within the cervix, the embryos may never reach the uterus to initiate pregnancy, and thus pregnancy rate may be reduced. As a result of the aforementioned problems, the pregnancy rate or liter size in embryo-transfer may be reduced. This results in annual monetary losses due to the cost of maintaining the non-pregnant recipient animals.

Not surprisingly, many varying techniques have been developed for the artificial insemination of livestock. The simplest and most common of these techniques is known as vaginal artificial insemination, or VAI. VAI has the advantage of being relatively inexpensive. VAI also requires little operator expertise or training. Unfortunately, VAI techniques are generally effective only when used in combination with relatively large amounts of freshly collected semen. In particular, VAI techniques have proven to be relatively ineffective when applied to sheep, especially when frozen semen is utilized.

Transcervical artificial insemination, or TAI, has been developed as an alternative to VAI techniques. When compared to VAI, TAI offers an alternative procedure for using frozen or fresh semen. TAI techniques also generally require fewer spermatozoa than VAI methods. Unfortunately, TAI techniques are more expensive and require more training than traditional VAI methods and present extremely variable results. Additionally, TAI techniques also present a risk of trauma to the subject animal.

Laparoscopic artificial insemination, or LAI, is another technique developed as an alternative to more traditional insemination techniques. In comparison to VAI, or TAI, LAI, offers the highest rate of pregnancy. LAI also requires the smallest number of spermatozoa per procedure. LAI is, however, an invasive and traumatic surgical procedure requiring a highly trained and licensed veterinarian. LAI also has the highest trauma risk potential.

In general, each of the preceding techniques has been applied to a number of differing types of livestock. For example, VAI, TAI and LAI methods been utilized for sheep as well as goat applications. It should be appreciated, however, that each of the preceding techniques may be more, or less, effective when utilized for a particular species. Practice has also shown that applications involving sheep are particularly problematic. In particular, female sheep, or ewes, have a cervical anatomy which includes four to six cervical rings. The rings function as partial seals for the cervical canal making traversal of the canal during an artificial insemination procedure problematic and often, ineffective. The presence of the cervical rings also increases the risk of traumatic injury during the artificial insemination procedure.

Other conventional artificial insemination (AI) techniques in the industry for some species may result in reduced pregnancy rate or litter size because not enough sperm cells were deposited into the uterus. To compensate for this and to maximize pregnancy rate or liter size, larger numbers of sperm cells are introduced than may be necessary if the entire insemination dose was deposited into the uterus. This is also due to the difficulty associated with passing a conventional straight AI apparatus through the cervix of some species.

A second difficulty associated with the artificial insemination of sheep is caused by chemical incompatibility between the cervical secretions of a ewe and cryoprotectants used to preserve spermatozoa. In more detail, it is generally the case that spermatozoa are combined with a cryoprotectant and frozen prior to implantation during an artificial insemination procedure. Freezing, of course, allows the spermatozoa to be stored for long periods of time without loss in potency. Freezing can only be accomplished, however, if a cryoprotectant is added to preserve the spermatozoa during the freezing process. Unfortunately, the cryoprotectants generally available are chemically incompatible with the chemical environment present in the cervix of a sheep. The resulting chemical reaction destroys the majority of the implanted spermatozoa defeating the object of the insemination procedure.

In light of the above, it is an object of the present invention to provide an apparatus and method for artificial insemination which minimizes the risk of trauma to the subject undergoing insemination. Another object of the present invention is to provide an apparatus and method for artificial insemination which minimizes the level of skill and training required for successful operation. Yet another object of the present invention to provide an apparatus and method for artificial insemination which maximizes the rate of successful insemination. Another object of the present invention to provide apparatus and method for artificial insemination which minimizes the amount of spermatozoa required for successful insemination. Another object of the present invention is to provide a non-surgical system and method for artificial insemination which is adaptable to the insemination of mammals for which there is presently not a highly successful insemination method. Still another object of the present invention is to provide apparatus and method for artificial insemination which is relatively simple to use, easy to manufacture, and cost effective.

### BRIEF SUMMARY

The present disclosure relates to an apparatus and a method useful for non-surgical impregnation of mammals. Specifically, a preferred embodiment for the present invention relates to transfer of fluid medium comprising semen or a fluid medium containing embryos into the uterus of an animal.

The present invention is characterized by the appended independent claim 1.

In accordance with one embodiment of the invention, an apparatus for depositing semen into a uterus of a mammal includes a non-linear probe portion that is curved to approximate the angle between the vagina and cervix of a mammal. The probe has a caudal end that is flared to accept a stopping device, the stopping device preventing airflow that would cause the lens of a camera to become contaminated. The probe has a plunger that passes through the length of the probe that, when activated, deposits the semen into the uterus of the mammal through an opening in the distal end of the probe. The distal end of the probe is equal to, or less than, the penis of a male of the same species that is being inseminated. The distal end having a material covering that is capable of receiving and holding a chemical that aids with the insemination process. In an embodiment of the present invention, the chemical is a mucus that is found on the penis of the male of the species, or the chemical equivalent of the mucus.

The distal portion is designed to carry a plastic straw (110) containing semen in a fluid extender. A plunger at the caudal end of the probe is depressed to dispel inseminate. The cervix of a sheep that has been poisoned by legumes is much more fibrous than the bovine cervix. Healthy sheep have no sinuous cervix, the present invention is of smaller diameter than the standard bovine artificial insemination gun (105). In an embodiment of the present invention, single use plastic sleeves are provided for the probe which have foam on their distal ends and are pre-saturated with synthetic ram mucus.

The species defense mechanism of a ewe has a number of activation triggers, and defensive responses. One response is the ewe's ability to rid herself of a foreign object that has managed to penetrate her cervix. Another possible defense response would be if the cervix has the ability to release spermicidal compounds.

A caudal camera and mount are provided that allow a user to guide the distal end of the probe to the cervix of the mammal being inseminated. The camera and mount generally comprise a swivel mount with tension adjustment knobs. The camera and mount are adjustable for different heights of artificial insemination technologies and different sizes of mammals. When in use, the camera lens resides a sufficient distance from the opening in the distal end to prevent contamination or fogging of the lens.

In accordance with another embodiment of the invention, a method of manufacture of an apparatus for of depositing semen or embryos into the uterus of a mammal includes the steps of providing a single-use plastic sleeve that fits over a curved artificial insemination probe, forming an area on the sleeve that is designed to retain a chemical such as the mucus from the penis of a male of the species, and pre-soaking the retention are of the sleeve with the mucus. Additional steps can include providing a probe that is curved to approximate the angle between the vagina and cervix of a mammal. A further step can include providing a camera that is inserted into the probe that allows the user to navigate the distal end of the probe into the cervix of a mammal.

A benefit of the present invention is that trans-cervical artificial insemination directly into the uterus of some species, the insemination time is reduced. Thus, labor costs are minimized. Still another benefit is that breeders can safely more quickly delivering the fluid to a multitude of animals and thus be more efficient.

These and other aspects and objects of the present invention will be better appreciated and understood when considered in conjunction with the following description and the accompanying drawings. It should be understood, however, that the following description, while indicating preferred embodiments of the present invention, is given by way of illustration and not of limitation. Many changes and modifications may be made within the scope of the present invention without departing from the spirit thereof, and the invention includes all such modifications.

### BRIEF DESCRIPTION OF THE FIGURES

FIG. 1 shows an artificial insemination gun that is curved to approximate the angle between the vagina and cervix of a mammal as according to an embodiment of the present invention.
Fig. 2 is a cross section of the caudal and non-tapered distal ends of an artificial insemination gun that is curved to approximate the angle between the vagina and cervix of a mammal as according to an embodiment of the present invention.
Fig. 3 is a cross-section of the caudal and tapered distal ends of a curved probe with a disposable sleeve attached as according to an embodiment of the present invention.

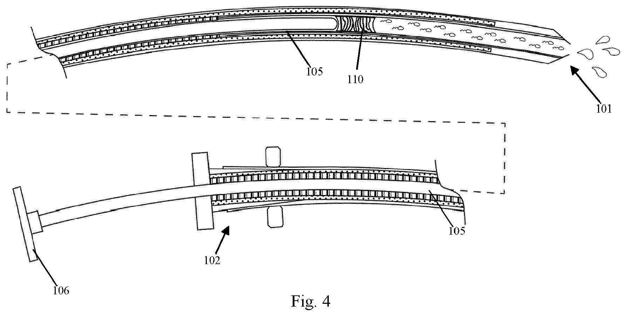

Fig. 4 is a cross-section of an artificial insemination gun dispensing inseminate as according to an embodiment of the present invention.

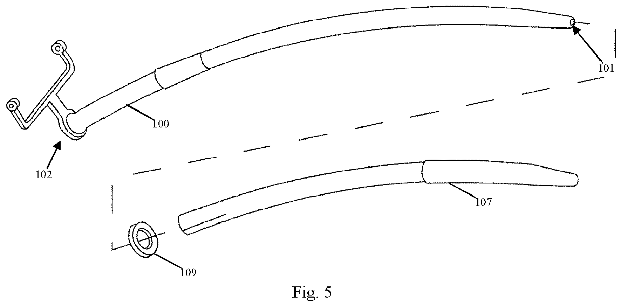

Fig. 5. is a diagram showing a disposable sleeve, probe and camera display mount along with an installation path of the sleeve onto probe as according to an embodiment of the present invention.

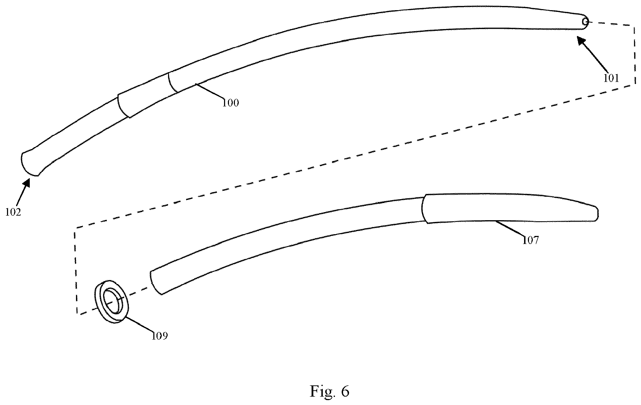

Fig. 6 is a diagram showing a disposable sleeve and probe along with an installation path of the sleeve onto probe as according to an embodiment of the present invention.

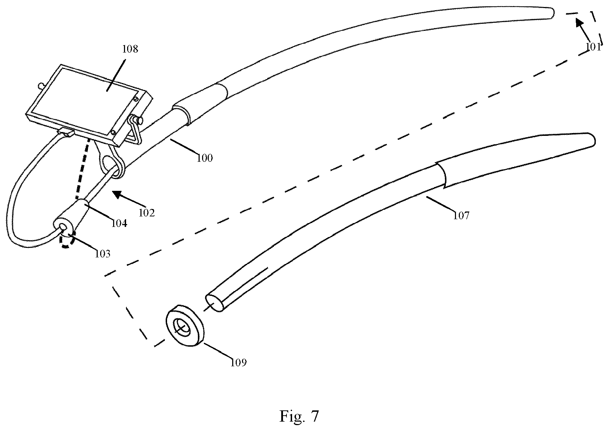

Fig. 7. is a diagram of a camera mount and camera cable inserted into an artificial insemination probe as according to an embodiment of the present invention.
Fig. 8 is a cross-sectional view of a probe being navigated through the ewe's cervix using a camera and display as according to an embodiment of the present invention.
Fig. 9 is a cross-sectional view of a ewe being inseminated as according to an embodiment of the present invention.

### DETAILED DESCRIPTION OF PREFERRED EMBODIMENTS

Referring now to the attached Figures, there are shown embodiments of an apparatus for the artificial insemination of mammals and a method of making the same. The apparatus comprises several components including a trans-cervical probe (100) that is curved to approximate the angle between the vagina and cervix of a mammal being inseminated. The trans-cervical probe (100) includes a long, narrow barrel portion having a distal end (101) and caudal end. The caudal end (102) is flared so that the terminal portion of the caudal end (102) is wider than the barrel of the trans-cervical probe (100). The flaring, as will be discussed further below, allows for a stopping device (103) to be easily inserted so that airflow through the barrel of the probe (100) is prevented. Stopping airflow provides several functional benefits that will become apparent in the remainder of this Description.

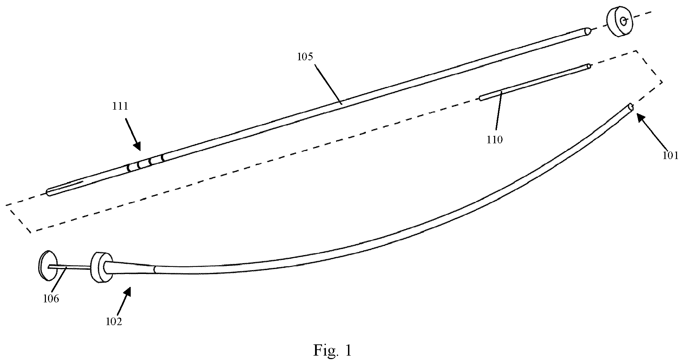

As illustrated in Fig. 1, the body of the trans-cervical probe (100) is curved. The curvature is introduced to the probe (100) so that the probe (100) has a shape that is approximately to the angle between the vagina and cervix of a mammal being inseminated. This allows the probe (100) to follow the path of the vagina and still place the distal end (101) of the probe (100) in the cervix at the appropriate orientation for delivery of inseminate, such as semen or sperm, into the uterus of the mammal being inseminated. The relative position of the distal end (101) can be gauged by observing marks (111) on the sheath of the artificial insemination gun. The marks (111) allow an operator to know when the distal end (101) is in the correct position for insertion of the inseminate material into a ewe's uterus. According to one embodiment the probe (100) has a curve of less than 45 degrees. The degree of curvature of the probe (100) may be expressed as the central angle to the ends of an arc or chord of a length, e.g. full length, of the probe (100).

Various sizes and shapes of the present invention are used to inseminate varying species of mammals. The invention does not rely on the probe (100) having specific dimensions or angles of curvature. Rather, each embodiment of the invention will have a different set of dimensions, all the dimensions intended shape the probe (100) so that it places the distal end (101) of the probe (100) at the cervix of the mammal in the proper orientation for insemination. In an embodiment of the present invention, a probe (100) used for the insemination of sheep is curved to approximate the angle between a ewe's vagina and cervix.

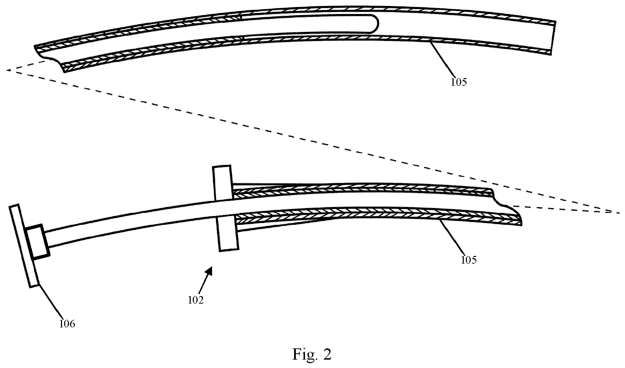

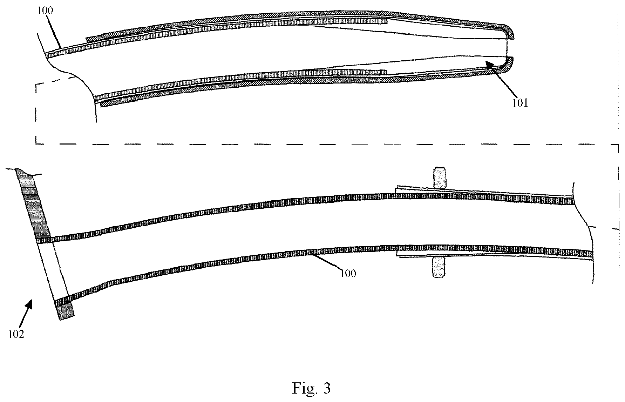

The internal structural details are readily appreciated in Figs 2 and 3 that show cross-sections of the caudal and distal ends (101) of the device. The caudal end (102) has a flared opening that allows for the insertion of an air stopping device (103) such as a rubber stopper. The stopping device (103) is shaped to form a tight seal with the caudal end (102) so that air cannot pass through the barrel of the probe (100) during insemination activities. If air could pass through the probe (100), then the lens of the camera that is used to navigate the tip of the probe (100) into the cervix could become contaminated by fluid or condensation entering the distal end (101) of the probe (100). Essentially, the air stopping device (103) is designed to block a sufficient amount of air from passing through the device that a buffer or protective pocket of air is formed immediately in front of the camera lens at the distal end (101) of the probe (100).

In some embodiments of the present invention, the stopping device (103) has an opening bored through the center of the device that allows a camera cable (104) to be passed through device and into the barrel of the probe (100). The bore extends linearly from one end of the device to the other and is of sufficiently small diameter to prevent air from escaping around the camera cable (104).

An insemination gun (105) is provided that is constructed to fit within the barrel of the probe (100). The gun (105) is further constructed to be inserted into the caudal end (102) of the probe (100), extend through the barrel of the probe (100), then contact and expel inseminate from a straw (110) or similar inseminate carrying device into the mammal when the plunger (106) at the non-distal end (101) of the gun (105) is depressed. The probe (100) is operationally configured to be inserted into the probe (100) after the camera is within drawn from the probe (100) and the probe (100) is in the proper position for insemination.

Also shown in Figs. 2-3 is the distal end (101) of the probe (100). During insemination procedures, the distal end (101) is guided through the mammal's vagina and through its cervix. The exact distance of extension through the cervix by the distal end (101) and into the uterus is specific to each embodiment of the invention. Some mammals may require the distal end (101) to pass further into the cervix for successful insemination than other mammals. Depending on the exact anatomy of the subject mammal, a larger mammal may require the distal end (101) of the probe (100) to extend further into the uterus than would be required for a smaller mammal. Each embodiment of the present invention will provide a distal end (101) that is constructed for the proper depth of insertion through the cervix and into the uterus for the mammal being inseminated.

To accomplish successful insemination, the present invention provides a covering (107) that is placed over the distal end (101) that is constructed of a material that can absorb a biochemical signature that is, or mimics, the biochemical signature of the male of the species. The covering (107) can be a porous material capable of absorbing fluid, mucus or the like, such as a foam or plastic. Prior to insemination, this porous covering (107) is placed over the distal end (101) of the probe (100) and secured in place by way of gripping adhesion to the exterior of the probe (100). In an embodiment of the present invention the covering (107) is single-use and comes preloaded with the biochemical signature. After insemination and the probe (100) is withdrawn from the animal, the covering (107) is designed to be easily removed from the probe (100) and disposed of.

The biochemical signature that is embedded within the porous covering (107) creates a chemical communication between the mammal and the probe (100) that simulates the chemical communication between the male and female mammals during mating. This communication, in some cases, serves to prevent the mammal being inseminated from rejecting the probe (100) or from self-defense mechanisms within the mammal from becoming active, such as the cervix of a sheep contracting or completely closing, which would prevent the distal end (101) of the probe (100) from entering the sheep's uterus. The biochemical signature can be the mucus extracted from the penis of a male of the species, a synthetic equivalent thereof, or any other chemical that aids in insemination of a mammal.

So that the porous covering (107) is not overly large for proper insemination, the distal end (101) of the probe (100) can be tapered down to a lesser size than the body of the probe (100). The exact length, amount of reduction of OD of the taper, and shape of the taper is, again, dependent on the specific embodiment. Different mammals may require the distal end (101) to be reduced to different diameters, may require a different length of tapering, and may require the distal end (101) to be tapered at different curvatures.

Some embodiments of the present invention provide a void area immediately behind the opening of the distal end (101). This void area can be an empty space within the tapered distal end (101) or can be an empty space within the porous covering (107) between the opening of the covering (107) and the opening of the distal end (101). Furthermore, the void area may be a combination of space in the porous opening and the distal end (101) of the probe (100). The void is designed to trap a pocket of air, when the aforementioned air stopper device is inserted, that reduces the amount of contamination that reaches the lens of the camera used to position the probe (100) during insertion and guidance of the probe (100) to the cervix and, or, uterus. Without the void area, there would be an increased probability that fluid or additional condensation would enter the probe (100) and obscure the lens.

The camera used to guide the distal end (101) of the probe (100) is inserted into the caudal end (102) of the probe (100) and extended to immediately behind the void area in the distal end (101) of the probe (100). The camera's lens can be at the end of a cable (104) that is passed through the barrel of the probe (100). The cable (104) is of sufficient flexibility to bend with the curvature of the probe (100) and not loose functionality. The viewscreen (108) can be attached to the probe (100) so that a user can observe the progress of probe (100) through the vagina and into the cervix.

### Method of Manufacture

The method for manufacturing the apparatus for artificially inseminating mammals comprises providing a probe (100) that is curved to approximate the angle between the vagina and cervix of a mammal. According to one embodiment the probe (100) has a curve of less than 45 degrees. Providing an air stopping device (103) that prevents the flow of air within the barrel of the probe (100) during insemination procedures. The air stopping device (103) being constructed so that it is removable from the probe (100) during the appropriate phase of artificial insemination. Constructing the caudal end (102) of the probe (100) to be flared so that it accepts the air stopping device (103). Constructing the barrel of the probe (100) so that it has an internal diameter that is sufficient to accept a camera cable (104) and an insemination gun (105). Constructing the barrel of the probe (100) so that it has an outer diameter that is sufficient to allow for passage of the probe (100) through the vagina of the mammal being inseminated to its cervix or uterus. Shaping the distal end (101) of the probe (100) so that it is tapered from a first outer diameter to a second outer diameter wherein the second outer diameter is less than the first outer diameter.

Providing a covering (107) for the distal end (101) of the probe (100) that can retain a biochemical signature of the male of the species of the mammal being inseminated. Constructing the covering (107) of a porous material such as a foam or a porous plastic or rubber mixture. Embedding the biochemical signature within the covering (107) prior to insemination procedures. Shaping the covering (107) so that it is easily attached over, then removed from, the distal end (101) of the probe (100). Creating the covering (107) to be a single-use device that is disposed of after insemination.

Creating a void area either within the distal end (101) of the probe (100), within the covering (107), or within a combination of the distal end (101) and the covering (107). The void area being of sufficient size to provide for a pocket of air that prevents some or all moisture, fluids, or condensation from reaching the lens of a camera that is used to properly position the probe (100) for insemination.

Providing a camera that displays an image on a viewscreen (108), the image being transmitted to the viewscreen (108) from a lens that can be mounted in the distal end (101) of the probe (100). Constructing the viewscreen (108) so that it is mountable to the probe (100) near the caudal end (102) of the probe (100). Providing a flexible lens member (104) that is extendable through the barrel of the curved probe (100) so that the lens is positioned prior to a void area within the probe (100) during insemination procedures.

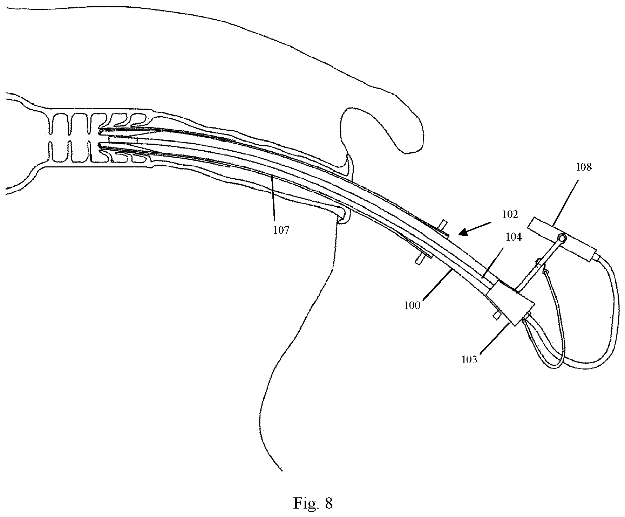

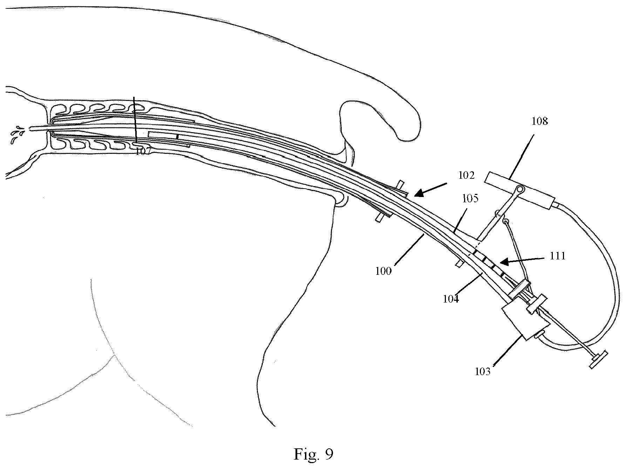

Figs. 8 and 9 show an embodiment of the present invention being used to artificially inseminate a ewe. In Fig 8, the probe (100) is being guided to the sheep's cervix through the use of a camera (104) that displays an image to an operator on the invention's display screen (108). The distal end of the probe passes through concentric rings within the ewe's cervix before reaching the uterus. Once it reaches the uterus, the air stopping device (103) is removed and the artificial insemination gun (105) passes through cervix and into the uterus. An amount of semen is deposited into the ewe's uterus. The distal end is able to pass through the cervix because of porous cover (107) that contains the biochemical signature compound. The compound prevents the ewe's self-defense mechanisms from activating during insemination.

In some embodiments of the present invention there are depth gauge marks (111) on the artificial insemination gun sheath. The depth gauge marks (111) are used to let an operator know the distal end (101) of the probe's position relative to a ewe's cervix. An operator can tell when the distal end (101) is in the cervix or through the cervix, and in proper position for insemination, by looking at the depth gauge marks.

Certain exemplary embodiments are further disclosed hereafter as clauses.
Clause 1: An apparatus for artificially inseminating mammals comprising a probe designed to trans-cervically deliver inseminate into the uterus of a mammal; the probe being curved to approximate the angle between the vagina and the cervix of the mammal; the probe having a distal end the distal end the distal end having an opening through which the inseminate is discharged into the mammal; the distal end being tapered and constructed to receive a covering; the covering being constructed of a porous material that is adapted to receive a biochemical signature; the covering being designed to cover at least the distal end of the probe; and the covering being embedded with the biochemical signature.
Clause 2: The apparatus of clause 1 further comprising an air stoppage device.
Clause 3: The apparatus of clause 1 or 2 further comprising a caudal end formed as part of the probe, the caudal end being flared to receive the air stoppage device.
Clause 4: The apparatus of any one of the preceding clauses further comprising constructing the distal end of the probe to create a void area.
Clause 5: The apparatus of any one of the preceding clauses further comprising constructing the covering of foam or a porous plastic.
Clause 6: The apparatus of any one of the preceding clauses further comprising constructing the covering so that it is disposable.
Clause 7: The apparatus of any one of the preceding clauses further comprising tapering the distal end of the probe.
Clause 8: The apparatus of any one of the preceding clauses wherein the biochemical signature is mucus collected from the penis of a male of the species that is being artificially inseminated.
Clause 9: An apparatus for artificially inseminating a mammal comprising: a probe having a barrel, a caudal, and a distal end; the probe being curved from the caudal end to the distal end the curvature of the probe approximating the angle between the vagina and the cervix of the mammal being artificially inseminated; the probe being hollow from the caudal end to the distal end the distal end having an opening through which inseminate is deposited into the uterus of the mammal being artificially inseminated; a covering that is designed to fit over the distal end of the probe, the covering being made of a porous material; the covering being embedded with a biochemical signature; and the biochemical signature being a chemical that simulates the chemical communication between a male mammal's penis and a female mammal's cervix.
Clause 10: The apparatus of clause 9 wherein the covering is constructed from a foam or a porous plastic.
Clause 11: The apparatus of clause 9 or 10 wherein the covering is constructed to extend beyond the distal end of the probe and over a portion of the barrel of the probe.
Clause 12: The apparatus of any one of the preceding clauses 9 to 11 further comprising a camera cable, lens, and viewscreen.
Clause 13: The apparatus of any one of the preceding clauses 9 to 12 wherein the camera cable is constructed to fit within the probe and can be curved in an angle that matches the curve of the probe.
Clause 14: The apparatus of any one of the preceding clauses 9 to 13 wherein the viewscreen is mounted to the caudal end of the probe.
Clause 15: The apparatus of any one of the preceding clauses 9 to 14 wherein the caudal end is flared.
Clause 16: The apparatus of any one of the preceding clauses 9 to 15 wherein the flare of the caudal end is shaped to receive an air stoppage device.
Clause 17: A method for manufacturing an apparatus for artificially inseminating a mammal comprising: providing a probe that is curved to approximate the angle between the vagina and cervix of a mammal; providing a distal end as part of the probe, forming a hole in the distal end through which inseminate is deposited into the uterus of the mammal; providing a covering that is designed to cover the distal end of the probe during artificial insemination procedures; forming a hole within and end of the cover through which inseminate is deposited into the uterus of the mammal; and embedding a biochemical signature within the cover.
Clause 18: The method of clause 17 further comprising forming a caudal end of the probe.
Clause 19: The method of claim 17 or 18 further comprising tapering the distal end of the probe from a first outer diameter to a second outer diameter.
Clause 20: The method of any one of the preceding clauses 17 to 19 further comprising constructing the cover from a foam or a porous plastic.
Clause 21: The method of any one of the preceding clauses 17 to 20 further comprising constructing the cover to be disposable.

While the particular device and method for manufacture for the device for artificial insemination as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as described in the appended claims.

The detailed description, above, forth numerous specific details to provide a thorough understanding of the present invention. However, those skilled in the art will appreciate that the present invention may be practiced without these specific details. In other instances, well known methods, procedures, components, and circuitry have not been described in detail to avoid obscuring the present invention.

All the references cited herein are incorporated by reference. The terms and expressions that have been employed in the foregoing specification are used as terms of description and not of limitation, and there is no intention, in the use of such terms and expressions, of excluding equivalents of the features shown and described or portions thereof, it being recognized that the scope of the invention is defined and limited only by the claims that follow.

## Claims

1. An apparatus for artificially inseminating mammals comprising a probe (100) designed to trans-cervically deliver inseminate into the uterus of a mammal, wherein:
- the probe (100) having a distal end (101) which comprises an opening through which the inseminate is discharged into the mammal;
- the distal end (101) of the probe (100) is tapered and constructed to receive a covering (107);
- the covering (107) is constructed of a porous material that is adapted to receive a biochemical signature;
- the covering (107) is designed to cover at least the distal end (101) of the probe (100); and
- the covering (107) is embedded with the biochemical signature.

2. The apparatus of claim 1 further comprising an air stoppage device (103).

3. The apparatus of claim 2 further comprising a caudal end formed as part of the probe (100), the caudal end (102) being flared to receive the air stoppage device (103).

4. The apparatus of claim 1 further comprising constructing the distal end (101) of the probe (100) to create a void area.

5. The apparatus of claim 1 wherein the covering is constructed from a foam or a porous plastic.

6. The apparatus of claim 1 further comprising constructing the covering (107) of foam or a porous plastic.

7. The apparatus of claim 1 further comprising constructing the covering (107) so that it is disposable.

8. The apparatus of claim 1 further comprising tapering the distal end (101) of the probe (100).

9. The apparatus of claim 1 wherein the biochemical signature is mucus collected from the penis of a male of the species that is being artificially inseminated.

10. The apparatus of claim 8 further comprising a camera cable (104), lens, and viewscreen (108).

11. The apparatus of claim 9 wherein the camera cable (104) is constructed to fit within the probe (100) and can be curved in an angle that matches the curve of the probe (100).

12. The apparatus of claim 10 wherein the viewscreen is mounted to the caudal end (102) of the probe (100).

13. The apparatus according to any one of the preceding claims, wherein the probe (100) is curved to approximate the angle between the vagina and the cervix of the mammal.

14. The apparatus of claim 13 wherein the probe (100) has a curve of less than 45 degrees.
